(19) **Europäisches Patentamt / European Patent Office / Office européen des brevets**

(11) **EP 4 613 855 A1**

(12) **EUROPEAN PATENT APPLICATION**
published in accordance with Art. 153(4) EPC

(43) Date of publication:
**10.09.2025 Bulletin 2025/37**

(21) Application number: **22964021.4**

(22) Date of filing: **04.11.2022**

(51) International Patent Classification (IPC):
*C12N 9/12* (2006.01)   *C12N 15/63* (2006.01)
*C12Q 1/686* (2018.01)

(52) Cooperative Patent Classification (CPC):
**C12N 9/12; C12N 15/63; C12Q 1/686**

(86) International application number:
**PCT/CN2022/129839**

(87) International publication number:
**WO 2024/092712 (10.05.2024 Gazette 2024/19)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicant: **BGI Shenzhen**
**Shenzhen, Guangdong 518083 (CN)**

(72) Inventors:
 • **ZHANG, Xiaohong**
 **Shenzhen, Guangdong 518083 (CN)**
 • **CAO, Ruyin**
 **Shenzhen, Guangdong 518083 (CN)**
 • **LIU, Xiaochen**
 **Shenzhen, Guangdong 518083 (CN)**

 • **CHEN, Xuemei**
 **Shenzhen, Guangdong 518083 (CN)**
 • **XIE, Qingqing**
 **Shenzhen, Guangdong 518083 (CN)**
 • **ZHENG, Yue**
 **Shenzhen, Guangdong 518083 (CN)**
 • **DONG, Yuliang**
 **Shenzhen, Guangdong 518083 (CN)**
 • **ZHANG, Wenwei**
 **Shenzhen, Guangdong 518083 (CN)**

(74) Representative: **Potter Clarkson**
**Chapel Quarter**
**Mount Street**
**Nottingham NG1 6HQ (GB)**

Remarks:
The complete document including Reference Table(s) and the Sequence Listing(s) can be downloaded from the EPO website

(54) **MMLV REVERSE TRANSCRIPTASE MUTANT**

(57)     Disclosed is an MMLV reverse transcriptase mutant. The present invention provides an MMLV reverse transcriptase mutant having mutations at amino acid residue positions 66 and 68 compared with the wild-type MMLV reverse transcriptase amino acid sequence set forth in SEQ ID NO. 2. The mutant retains the reverse transcriptase activity. The mutant features improved template conversion performance, improved non-template base addition performance, or improved thermal resistance. According to the present invention, MMLV reverse transcriptase mutants with improved template conversion performance compared with the wild type and improved non-template base addition performance or thermal stability are selected. The selected mutants are suitable for RNA sequencing using library preparation methods based on template conversion, such as Smart-Seq, nanopore sequencing, and 5' RACE, and can also be applied to RT-qPCR and the like.

EP 4 613 855 A1

**Description**

**FIELD**

The present disclosure belongs to the field of enzyme engineering, and particularly relates to an MMLV reverse transcriptase mutant.

**BACKGROUND**

Reverse transcriptase is a special DNA polymerase, also called RNA-dependent DNA polymerase, which can synthesize DNA using RNA as a template. Reverse transcriptase was first found in RNA viruses. Reverse transcriptases derived from RNA viruses are currently the most deeply studied and widely applied reverse transcriptases, mainly including Moloney murine leukemia virus reverse transcriptase (MMLV RT), human immunodeficiency virus reverse transcriptase (HIV RT), and avian myeloblastosis virus reverse transcriptase (AMV RT).

In the study of RNA, RNA is usually converted into a more stable complementary DNA (cDNA) by reverse transcription, and then studied and analyzed by cloning, PCR, gene expression chip, sequencing, and other technologies. Therefore, reverse transcription is a critical step in the workflow of many RNA experiments.

With the rapid development of sequencing technology, RNA-Seq has become the main technical means for studies such as whole transcriptome analysis, gene differential expression, and mRNA variable splicing. Single-cell transcriptome sequencing (scRNA-Seq) and spatial transcriptome sequencing have also emerged gradually in recent years. Single-cell RNA sequencing is a technology of sequencing the transcriptome at the single-cell level, which can study gene expression in a single cell and solve the problem of cellular heterogeneity that cannot be solved by tissue sample sequencing, making it possible to analyze the behavior and mechanism of a single cell and its relationship with the body, and can be applied to the research fields such as the human cell atlas construction, tumor heterogeneity study, immunity, development, and differentiation. Although conventional transcriptome sequencing and scRNA-Seq can provide detailed data on tissues or cell populations, it is difficult to capture the spatial information of the cells, thus, the relationship between cell environment and gene expression cannot be accurately obtained. In contrast, spatial transcriptome sequencing makes it possible to explore gene expression in the context of tissue microenvironment. Spatial transcriptome sequencing can combine the spatial information of RNA with morphological content to draw gene expression maps, providing important information about the relationship between cell function, phenotype, and location in the tissue microenvironment.

In addition, the template-switching-based library preparation method has become the preferred method for single-cell transcriptome sequencing and spatial transcriptome sequencing due to its characteristics of simple operation and low RNA consumption. In addition to utilizing the polymerization performance of reverse transcriptase with RNA as a template, two other properties of reverse transcriptase are also utilized in the template switching-base library preparation method, i.e., non-templated addition (NTA) and template switching (TS). In the template-switching-based library preparation, reverse transcription is first initiated at the 3' end of mRNA using Oligo(dT) fused with a known sequence as a primer, and non-template-dependent bases (commonly known as CCC) are then added to the 3' end of cDNA through the NTA activity of reverse transcriptase. The protruding non-template-dependent CCC bases can bind to the rGrGrG at the 3' end of template switching oligo (TSO). At this time, the reverse transcriptase plays its template switching performance to switch from RNA as a template to TSO as a template for the extension, ultimately forming a cDNA with the known sequence at both ends, which can be used as a template for PCR amplification or library construction.

At present, the reverse transcriptase used in single-cell transcriptome sequencing, spatial transcriptome sequencing, Smart-Seq, and the like is mainly modified MMLV reverse transcriptase derived from Moloney murine leukemia virus. The modified MMLV reverse transcriptases generally have high reverse transcriptase activity, reduced RNase H activity, or high-temperature resistance, however, there are few modifications of the non-templated base addition performance and template switching performance required for template-switching-based library preparation methods. Although the modified reverse transcriptases have been commercialized and applied to a variety of RNA sequencing, they still have shortcomings, such as low cDNA yield and low gene capture, which limit the development of transcriptome sequencing technology to some extent. Therefore, it is crucial to develop a reverse transcriptase with higher template switching performance. activity

**SUMMARY**

It is an object of the present disclosure to provide an MMLV reverse transcriptase mutant with improved template switching performance, improved non-templated base addition performance, or improved thermal resistance.

In a first aspect, the present disclosure provides a Moloney murine leukemia virus, MMLV reverse transcriptase mutant having mutations at amino acid residue sites 66 and 68 compared with an amino acid sequence of a wild-type MMLV reverse transcriptase as set forth in SEQ ID NO. 2, the MMLV reverse transcriptase mutant retaining the reverse transcriptase activity.

In the above, sites 66 and 68 are site 66 and site 68 of SEQ ID NO. 2. The mutations above are point mutations that do not result in insertions, deletions, or frameshifts.

In the above, for the mutant, M at site 66 is mutated to V, K, Y, or L, and Q at site 68 is mutated to N or K.

In a second aspect, the present disclosure provides an MMLV reverse transcriptase mutant, wherein one or more of the following amino acid residue sites of the mutant are mutated on the basis of the mutant described in the first aspect: 39, 62, 65, 67, 69, 70, 80, 81, 99, 105, 109, 116, 124, 152, 175, 176, 186, 200, 269, 284, 286, 289, 302, 306, 313, 333, 334, 425, 435, 450, 454, 524, 562, 583, and 653.

In the above, sites 39, 62, 65, 67, 69, 70, 80, 81, 99, 105, 109, 116, 124, 152, 175, 176, 186, 200, 269, 284, 286, 289, 302, 306, 313, 333, 334, 425, 435, 450, 454, 524, 562, 583, and 653 are sites of the mutant, which are consistent with the positions of sites 39, 62, 65, 67, 69, 70, 80, 81, 99, 105, 109, 116, 124, 152, 175, 176, 186, 200, 269, 284, 286, 289, 302, 306, 313, 333, 334, 425, 435, 450, 454, 524, 562, 583, and 653 of SEQ ID NO. 2.

In the above, one or more mutated sites are respectively mutated in the way:

M at site 39 is mutated to D or R;
K at site 62 is mutated to R;
P at site 65 is mutated to N, T, K, or L;
S at site 67 is mutated to T;
E at site 69 is mutated to K or R;
A at site 70 is mutated to S;
R at site 80 is mutated to T;
L at site 81 is mutated to A;
L at site 99 is mutated to A;
G at site 105 is mutated to R;
Y at site 109 is mutated to R;
R at site 116 is mutated to D;
D at site 124 is mutated to K;
K at site 152 is mutated to R;
P at site 175 is mutated to S;
E at site 176 is mutated to R;
T at site 186 is mutated to A;
D at site 200 is mutated to N;
L at site 269 is mutated to R;
R at site 284 is mutated to T;
E at site 286 is mutated to R;
M at site 289 is mutated to L;
E at site 302 is mutated to K;
T at site 306 is mutated to R;
W at site 313 is mutated to F;
L at site 333 is mutated to A or K;
F at site 334 is mutated to N;
K at site 425 is mutated to R;
L at site 435 is mutated to G;
R at site 450 is mutated to H;
N at site 454 is mutated to K;
D at site 524 is mutated to G or A;
E at site 562 is mutated to Q;
D at site 583 is mutated to N;
D at site 653 is mutated to N.

The MMLV reverse transcriptase mutant provided in the first or second aspect above has the following characteristics. The MMLV reverse transcriptase mutant has improved template switching activity, improved non-templated base addition performance, and/or improved thermal stability compared with the wild-type MMLV reverse transcriptase.

In some embodiments of the present disclosure, the MMLV reverse transcriptase mutant provided in the first or second aspect above further has the following characteristics. Compared with the wild-type MMLV reverse transcriptase, the template switching activity of the MMLV reverse transcriptase mutant is increased to 1 to 42 times that of the wild-type MMLV reverse transcriptase.

In some embodiments of the present disclosure, the MMLV reverse transcriptase mutant provided in the first or second aspect above further has the following characteristics. Compared with the wild-type MMLV reverse transcriptase, the non-templated base addition performance of the MMLV reverse transcriptase mutant is increased to 1 to 1.85 times that of the wild-type MMLV reverse transcriptase.

In some embodiments of the present disclosure, the MMLV reverse transcriptase mutant provided in the first or second aspect above has any one of the following mutations:

double-site mutations: M66L and Q68K; or, M66V and Q68K; or

three-site mutations: M66V, S67T, and Q68K; or, M66V, Q68K, and R80T; or, M66V, Q68K, and D124K; or, M66V, Q68K, and D200N; or, M66V, Q68K, and T306K; or, M66V, Q68K, and T306R; or

four-site mutations: M66V, Q68K, E176R, and L333A; or

six-site mutations: M66L, Q68K, E69K, E302K, W313F, and N454K; or, M66V, Q68K, E69K, E302K, W313F, and N454K; or

seven-site mutations: M66V, Q68K, E69K, E302K, T306R, W313F, and N454K; or, M66L, Q68K, E69K, E302K, W313F, N454K, and D524G; or, M66V, Q68K, E69K, E302K, W313F, N454K, and D524G; or

eight-site mutations: M66L, Q68K, E69K, E302K, W313F, N454K, D524G, and D653N; or, M66V, Q68K, E69K, E302K, W313F, N454K, D524G, and D653N; or

nine-site mutations: M66V, Q68K, E69K, E302K, T306R, W313F, N454K, D524G, and D653N.

In some embodiments of the present disclosure, the MMLV reverse transcriptase mutant with improved template switching activity (the template switching performance is increased to 1 to 42 times that of the wild-type MMLV reverse transcriptase) is a mutant having any one of the following mutant forms:

two-site mutations: M66L and Q68K; or, M66V and Q68K; or

three-site mutations: M66V, S67T, and Q68K; or, M66V, Q68K, and R80T; or, M66V, Q68K, and D124K; or, M66V, Q68K, and D200N; or, M66V, Q68K, and T306K; or, M66V, Q68K, and T306R; or

four-site mutations: M66V, Q68K, E176R, and L333A; or

eight-site mutations: M66L, Q68K, E69K, E302K, W313F, N454K, D524G, and D653N; or, M66V, Q68K, E69K, E302K, W313F, N454K, D524G, and D653N; or

nine-site mutations: M66V, Q68K, E69K, E302K, T306R, W313F, N454K, D524G, and D653N.

In some embodiments of the present disclosure, the MMLV reverse transcriptase mutant with improved template switching activity compared with commercial SSII enzyme is a mutant having any one of the following mutant forms:

two-site mutations: M66L and Q68K; or, M66V and Q68K; or

three-site mutations: M66V, S67T, and Q68K; or, M66V, Q68K, and D124K; or, M66V, Q68K, and D200N; or, M66V, Q68K, and T306R; or

four-site mutations: M66V, Q68K, E176R, and L333A; or

eight-site mutations: M66L, Q68K, E69K, E302K, W313F, N454K, D524G, and D653N; or, M66V, Q68K, E69K, E302K, W313F, N454K, D524G, and D653N; or

nine-site mutations: M66V, Q68K, E69K, E302K, T306R, W313F, N454K, D524G, and D653N.

In a third aspect, the present disclosure provides a nucleic acid molecule encoding the mutant provided in the first or second aspect above.

In a fourth aspect, the present disclosure provides an expression cassette, recombinant vector, or transgenic cell line including the nucleic acid molecule described in the third aspect above.

In some embodiments of the present disclosure, the recombinant vector is a vector obtained by cloning the nucleic acid molecule described in the third aspect above into an expression vector. The expression vector may be pET-22b or other prokaryotic expression vectors.

In an embodiment of this aspect, the mutants in various recombinant vectors are all expressed under the promoter of pET-22b and fused with six His tags at the C-terminus. The His-tags can be used for Ni-column affinity purification during purification.

In a fifth aspect, the present disclosure provides a recombinant bacterium including the nucleic acid molecule described in the third aspect above.

In some embodiments of the present disclosure, the recombinant bacterium is a recombinant bacterium obtained by transferring the nucleic acid molecule described above into a host cell, which may be *E. coli,* such as *E. coli* BL21.

In a sixth aspect, the present disclosure provides the use of the nucleic acid molecule described in the third aspect, or the expression cassette, recombinant vector, or transgenic cell line described in the fourth aspect, or the recombinant bacterium described in the fifth aspect in the preparation of MMLV reverse transcriptase.

In a seventh aspect, the present disclosure provides a method for preparing the mutant described in the first or second aspect, including the following steps: culturing the recombinant bacterium described in the fifth aspect, and performing an induction treatment, to obtain the mutant.

In some embodiments of the present disclosure, the method specifically includes the following steps: culturing the recombinant bacterium to an $OD_{600}$ between 0.6 and 0.8, adding IPTG as an inducer at a final concentration of 0.5 mM for induction culture, collecting the inductioncultured bacteria, ultrasonically crushing the bacteria, and then purifying to obtain the mutant.

In some embodiments of the present disclosure, the purification may be crude purification, specifically achieved using a His MultiTrap HP purification plate. Alternatively, the purification may be fine purification, including affinity chromatography, anion exchange chromatography, and cation exchange chromatography.

In an eighth aspect, the present disclosure provides a kit including the mutant described in the first or second aspect.

In the above, the kit further includes at least one of one or more nucleotides, one or more DNA polymerases, one or more primers, and one or more terminators.

In some embodiments of the present disclosure, the kit further includes one or more buffers.

In a ninth aspect, the present disclosure provides a method for reverse transcription of a nucleic acid molecule, including the following steps:

mixing at least one nucleic acid template with at least one reverse transcriptase to obtain a mixture, wherein the reverse transcriptase is the mutant described in the first or second aspect above; and
performing a reverse transcription reaction of the mixture to obtain a reverse transcription nucleic acid molecule that is entirely or partially complementary to at least one template.

In a tenth aspect, the present disclosure provides a method for amplifying a nucleic acid, including the following steps:

performing a first mixing reaction by mixing at least one nucleic acid template with at least one reverse transcriptase, to obtain a reaction product, wherein the reverse transcriptase is the mutant described in the first or second aspect above; and
performing a second mixing reaction by mixing the reaction product with at least one DNA polymerase, to obtain an amplified nucleic acid molecule.

In an eleventh aspect, the present disclosure provides a method for constructing a template-switching-based sequencing library, including the following steps:

1) extracting RNA from a biological sample to be tested and performing a reverse transcription by using the method described in the ninth aspect above to obtain a cDNA; and
2) constructing a sequencing library based on the cDNA.

In the above, the biological sample to be tested is animal tissue, plant tissue, bacteria, or cells.

In the above, the biological sample to be tested is selected from at least one of soil, feces, blood, and serum.

In the above, the sequencing library is a high-throughput sequencing library.

The present disclosure relates to reverse transcriptase with improved performances, including improved template switching performance, improved non-templated base addition performance, or improved thermal resistance, which can be applied to RT-PCR technology, first cDNA strand synthesis, RNA sequencing, 5' RACE technology, and the like.

The MMLV reverse transcriptase mutant of the present disclosure has significantly improved template switching performance, non-templated base addition performance, or thermal stability compared with the wild type, and significantly improved cDNA yield in the template-switching-based library preparation, and is particularly suitable for RNA sequencing using template-switching-based library preparation methods, such as Smart-Seq and spatial transcriptome sequencing.

The present disclosure is further described below with reference to examples.

## BRIEF DESCRIPTION OF DRAWINGS

Fig. 1 is a flow chart for screening the template switching performance of MMLV reverse transcriptase and its mutants.

Fig. 2 shows an example of capillary electrophoresis for screening the template switching performance of MMLV reverse transcriptase and its mutants.

Fig. 3 shows the test results of the thermal stability of MMLV reverse transcriptase.

Fig. 4 is a schematic diagram of one of template-switching-based library construction methods (Smart-Seq).

Fig. 5 shows a correlation analysis of the effects and various performances of MMLV reverse transcriptase and its mutants in template-switching-based library construction methods.

Fig. 6 is a schematic diagram of a method for verifying the template switching performance of MMLV reverse transcriptase and its mutants.

Fig. 7 shows the verification of the template switching performance of fine-purified MMLV reverse transcriptase and its mutants.

Fig. 8 shows the visualization results of the STOmics assay for commercially available MMLV reverse transcriptase and fine-purified MMLV reverse transcriptase mutants.

Fig. 9 shows the comparative data of Median Gene Type and Median MID of commercially available MMLV reverse transcriptase and the modified and screened MMLV reverse transcriptase mutant Clone 79.

## DESCRIPTION OF EMBODIMENTS

According to the present disclosure, by analyzing the structure of MMLV reverse transcriptase and exploring sites that may be associated with template switching performance, non-templated base addition, and thermal stability, plasmids containing coding sequences for MMLV reverse transcriptase mutants are constructed by using genetic engineering means for the explored sites. The plasmids are transferred into *E. coli* to express MMLV reverse transcriptase. Then the protein is purified by protein chromatography technology to obtain MMLV reverse transcriptase mutants. Specifically, plasmids containing sequences encoding different MMLV reverse transcriptase mutants are constructed by means of site-directed mutagenesis PCR; see Example 1 for details. Then, the constructed plasmids containing sequences encoding different MMLV reverse transcriptase mutants are transferred into *E. coli* for culture and induction expression. Finally, MMLV reverse transcriptase is purified and extracted by protein chromatography to obtain MMLV reverse transcriptase containing different mutations; see Example 2 for details.

Hereinafter, the embodiments of the present disclosure will be described in detail with reference to examples. However, those skilled in the art will understand that the following examples are only intended to illustrate the present disclosure and should not be construed as limiting the scope of the present disclosure. All the reagents or instruments used without specifying manufacturers are all conventional commercially products which are commercially available. The experimental methods used are conventional methods unless otherwise specified.

V/V in the following examples represents the volume ratio.

SSII, in the following examples, is the commercial enzyme SuperScript II (Invitrogen, Cat. No. 1806471).

## Example 1. Construction of expression plasmids of MMLV reverse transcriptase and its mutants

I. Wild-type MMLV reverse transcriptase and an expression plasmid expressing the enzyme

The expression plasmid pET-22b-MMLV containing the gene encoding wild-type MMLV reverse transcriptase was purchased from Genscript Biotech Corporation, which is a plasmid obtained by cloning the gene encoding wild-type MMLV reverse transcriptase into the pET-22b vector.

The pET-22b-MMLV expresses a fusion protein of wild-type MMLV reverse transcriptase with six His tags fused to the C-terminus. The His-tag was used for protein purification.

The gene sequence of the wild-type MMLV reverse transcriptase is SEQ ID NO. 1, and the amino acid sequence encoded by it is SEQ ID NO. 2.

II. Preparation of plasmids expressing MMLV reverse transcriptase mutants

Plasmids expressing MMLV reverse transcriptase mutants were constructed by site-directed mutagenesis. Specifically, forward and reverse mutation primer pairs were designed based on the mutation sites screened by rational design, and PCR was performed using pfu DNA polymerase. The PCR reaction system consisted of 1x Pfu buffer (containing MgSO$_4$), 0.2 mM dNTPs, 0.5 $\mu$M forward primer, 0.5 $\mu$M reverse primer, 0.05 U/$\mu$L Pfu DNA polymerase, and 1 ng/$\mu$L template plasmid pET-22b-MMLV. The PCR program was as follows: pre-denaturation at 95°C for 3 min, 18 cycles of denaturation at 95°C for 30 s, annealing at 55°C for 30 s, extension at 68°C for 8 min, and final extension at 68°C for an additional 10 min. After the reaction, DpnI was added at a final concentration of 0.4 U/$\mu$L, and digestion was performed at 37°C for 1 h. Then, 5 $\mu$L of the digested product was used to transform *E. coli* competent cells DH5$\alpha$. The next day, a single clone was picked from the plate and cultured, and then the plasmid was extracted and analyzed by sequencing and alignment to determine whether the obtained mutant was correct.

The plasmids expressing MMLV reverse transcriptase mutants were the plasmids obtained by replacing the gene encoding wild-type MMLV reverse transcriptase in the pET-22b-MMLV vector with the gene encoding MMLV reverse transcriptase mutant.

Each MMLV reverse transcriptase mutant was obtained by mutating the wild-type MMLV reverse transcriptase as set forth in SEQ ID NO. 2 according to the mutation sites and modes shown in second column of Table 1 below, with other amino acid residues unchanged.

The encoding gene of each MMLV reverse transcriptase mutant was obtained by mutating the encoding gene of wild-type MMLV reverse transcriptase as set forth in SEQ ID NO. 1 according to the codons corresponding to the mutation sites and the corresponding mutation modes shown in the second column of Table 1 below, with other nucleotides unchanged.

The information on the mutants is shown in Table 1.

Table 1. Information on MMLV reverse transcriptase mutants

| Mutant name | Mutation sites and modes |
| --- | --- |
| WT | - |
| Clone 1 | M39D |
| Clone 2 | M39R |
| Clone 3 | K62R |
| Clone 4 | P65N |
| Clone 5 | P65T |
| Clone 6 | P65K |
| Clone 7 | P65L |
| Clone 8 | M66V |
| Clone 9 | M66K |
| Clone 10 | M66Y |
| Clone 11 | Q68N |
| Clone 12 | E69K |
| Clone 13 | E69R |
| Clone 14 | A70S |
| Clone 15 | R80T |
| Clone 16 | L81A |
| Clone 17 | L99A |
| Clone 18 | G105R |
| Clone 19 | Y109R |
| Clone 20 | R116D |
| Clone 21 | D124K |
| Clone 22 | K152R |
| Clone 23 | P175S |
| Clone 24 | E176R |
| Clone 25 | T186A |
| Clone 26 | L269R |
| Clone 27 | R284T |
| Clone 28 | M289L |

(continued)

| Mutant name | Mutation sites and modes |
|---|---|
| Clone 29 | T306R |
| Clone 30 | W313F |
| Clone 31 | L333A |
| Clone 32 | L333K |
| Clone 33 | F334N |
| Clone 34 | K425R |
| Clone 35 | L435G |
| Clone 36 | N454K |
| Clone 37 | D524G |
| Clone 38 | D524A |
| Clone 39 | E562Q |
| Clone 40 | D583N |
| Clone 41 | M66L, S67T |
| Clone 42 | M66V, S67T |
| Clone 43 | M66L, Q68K |
| Clone 44 | M66V, Q68K |
| Clone 45 | E302K, E286R |
| Clone 46 | D524G, R450H |
| Clone 47 | D524G, E562Q |
| Clone 48 | E562Q, D583N |
| Clone 49 | M66V, S67T, Q68K |
| Clone 50 | M66V, Q68K, R80T |
| Clone 51 | M66V, Q68K, D124K |
| Clone 52 | M66V, Q68K, D200N |
| Clone 53 | M66V, Q68K, T306K |
| Clone 54 | M66V, Q68K, T306R |
| Clone 55 | E286R, E302K, D524A |
| Clone 56 | M66V, Q68K, E176R, L333A |
| Clone 57 | M39D, E69K, E302K, W313F, N454K, D524G, D653N |
| Clone 58 | M39R, E69K, E302K, W313F, N454K, D524G, D653N |
| Clone 59 | K62R, E69K, E302K, W313F, N454K, D524G, D653N |
| Clone 60 | E69K, R80T, E302K, W313F, N454K, D524G, D653N |
| Clone 61 | E69K, L99A, E302K, W313F, N454K, D524G, D653N |
| Clone 62 | E69K, G105R, E302K, W313F, N454K, D524G, D653N |
| Clone 63 | E69K, Y109R, E302K, W313F, N454K, D524G, D653N |
| Clone 64 | E69K, D124K, E302K, W313F, N454K, D524G, D653N |
| Clone 65 | E69K, K152R, E302K, W313F, N454K, D524G, D653N |
| Clone 66 | E69K, D200N, E302K, W313F, N454K, D524G, D653N |
| Clone 67 | E69K, E286K, E302K, W313F, N454K, D524G, D653N |

(continued)

| Mutant name | Mutation sites and modes |
|---|---|
| Clone 68 | E69K, E286R, E302K, W313F, N454K, D524G, D653N |
| Clone 69 | P65K, E69K, E302K, W313F, N454K, D524G, D653N |
| Clone 70 | P65N, E69K, E302K, W313F, N454K, D524G, D653N |
| Clone 71 | P65T, E69K, E302K, W313F, N454K, D524G, D653N |
| Clone 72 | M66L, S67T, E69K, E302K, W313F, N454K, D524G, D653N |
| Clone 73 | M66V, E69K, E302K, W313F, N454K, D524G, D653N |
| Clone 74 | M66L, Q68K, E69K, E302K, W313F, N454K, D524G, D653N |
| Clone 75 | M66V, Q68K, E69K, E302K, W313F, N454K, D524G, D653N |
| Clone 76 | E69K, A70S, E302K, W313F, N454K, D524G, D653N |
| Clone 77 | E69K, E302K, T306K, W313F, N454K, D524G, D653N |
| Clone 78 | E69K, E302K, T306R, W313F, N454K, D524G, D653N |
| Clone 79 | M66V, Q68K, E69K, E302K, T306R, W313F, N454K, D524G, D653N |
| Clone 80 | M66Y, E69K, E302K, W313F, N454K, D524G, D653N |
| Clone 81 | E69K, P175S, E302K, W313F, N454K, D524G, D653N |
| Clone 82 | E69K, E176R, E302K, W313F, N454K, D524G, D653N |
| Clone 83 | E69K, E302K, W313F, L333K, N454K, D524G, D653N |
| Clone 84 | E69K, E302K, W313F, L333A, N454K, D524G, D653N |
| Clone 85 | E69K, E176R, E302K, W313F, L333K, N454K, D524G, D653N |
| Clone 86 | E69K, E176R, E302K, W313F, L333A, N454K, D524G, D653N |
| Clone 87 | E69K, E302K, W313F, N454K, D524G, D653N |

In the table above, WT represents the wild-type MMLV reverse transcriptase as set forth in SEQ ID NO. 2; the second column shows the mutation sites and modes on the basis of the wild-type MMLV reverse transcriptase as set forth in SEQ ID NO. 2 when mutating.

**Example 2. Expression and purification of MMLV reverse transcriptase and its mutants**

Wild-type MMLV reverse transcriptase and each mutant shown in Table 1 were expressed under the control of the of pET-22b promoter and fused with six His tags at the C-terminus. The His-tags could be used for Ni-column affinity purification during purification.

The expression and purification of the wild-type MMLV reverse transcriptase and each mutant shown in Table 1 included both small-scale expression and crude purification, and large-scale expression and fine purification. The former is used for screening MMLV reverse transcriptase mutants, and the latter is used for the determination and analysis of other performances of MMLV reverse transcriptase.

1. Small-scale expression and crude purification

Small-scale expression and crude purification of the wild-type MMLV reverse transcriptase and its mutants were carried out as follows. The pET-22b-MMLV plasmid expressing the wild-type MMLV reverse transcriptase and the plasmids expressing various MMLV reverse transcriptase mutants were transferred into BL21 competent cells, respectively. Then, a single colony was picked into 15 mL of LB medium containing Amp resistance (100 $\mu$g/mL) and cultured at 37°C with shaking at 200 rpm/min to an $OD_{600}$ between 0.6 and 0.8. The inducer IPTG was added at a final concentration of 0.5 mM and then cultured at 16°C and 200 rpm/min for 18 h to induce protein expression. Finally, the induced bacteria were collected by centrifugation at 8000 rpm/min for 5 min. 500 $\mu$L of MMLV reverse transcriptase Ni column A solution (50 mM Tris, 500 mM NaCl, 5% (V/V) glycerol, 10 mM imidazole, the balance is water, pH 7.5) was added to the collected bacteria for resuspension. Non-contact batch ultrasound was performed under an ice-water bath condition, with ultrasound intensity of 70% and continuous ultrasound for 30 min. The supernatant was then collected by centrifugation at 13000 rpm and 4°C for 10 min. The supernatant was added to the wells of the balanced His MultiTrap HP purification plate (purchased from GE). After standing for 15 min, the solution was removed using a vacuum pressure device. 2 mL of MMLV reverse transcriptase Ni column A solution was added for rinsing, and the solution was then removed using a vacuum pressure device. A 96-well plate was placed below the His MultiTrap HP purification plate and fixed well. Then, 100 μL of MMLV reverse transcriptase Ni column B solution (50 mM Tris, 500 mM NaCl, 5% (V/V) glycerol, 500 mM imidazole, the balance was water, pH 7.5) was added. The eluted proteins were collected by centrifugation at 500 g for 2 min using a centrifuge. 100 μL of glycerol was added to the sample wells of the 96-well plate, mixed well, transferred to 1.5 mL centrifuge tubes, and stored at -20°C to obtain crude-purified wild-type MMLV reverse transcriptase and its various mutants.

2. Large-scale expression and fine purification

Large-scale expression and fine purification of the MMLV reverse transcriptase and its mutants were carried out as follows.

The pET-22b-MMLV plasmid expressing the wild-type MMLV reverse transcriptase and the plasmid expressing each MMLV reverse transcriptase mutants was transformed into BL21 competent cells, respectively. Then, a single colony was picked into 15 mL of LB medium containing Amp resistance (100 μg/mL) and cultured at 37°C and 200 rpm/min overnight. The next day, the culture was transferred to 1500 mL of fresh LB medium containing Amp resistance (100 μg/mL) at a ratio of 1:100 and cultured at 37°C with shaking at 200 rpm/min to an $OD_{600}$ between 0.6 and 0.8. The inducer IPTG was added at a final concentration of 0.5 mM and then cultured at 16°C and 200 rpm/min for 18 h to induce protein expression. Finally, the induced bacteria were collected by centrifugation at 8000 rpm/min for 10 min.

The fine purification of the MMLV reverse transcriptase and its mutants includes affinity chromatography, anion exchange chromatography, and cation exchange chromatography. Specifically, the induced bacteria collected above were weighed, resuspended in a ratio of 10 mL MMLV reverse transcriptase Ni column A solution pre 1 g of bacteria, and subjected to ultrasonication under an ice-water bath condition. The ultrasound conditions were as follows: the diameter of the horn was10 mm, the ultrasonic intensity was 40%, ultrasound treatment lasted for 2 s, the interval lasted for 3 s, and ultrasound treatment for 30 min. The supernatant was then collected by centrifugation at 13000 rpm and 4°C for 30 min.

The samples prepared in the previous step were subjected to affinity purification. The specific steps were as follows.

According to the AKTA operating procedures, the working pump and system were rinsed with filtered and degassed MillQ water and connected with pre-packed column HisTrap FF 5 mL at a flow rate of 0.5 mL/min. The column was rinsed with 5CV of $H_2O$ and then balanced with 5CV of MMLV reverse transcriptase Ni column A solution. Then, the supernatant collected in the previous step was loaded onto the chromatographic column at 5 mL/min. After loading, the column was rinsed with 20CV of MMLV reverse transcriptase Ni column A solution, and then eluted with a linear gradient of 0-100% (10CV) of MMLV reverse transcriptase Ni column B solution and the target proteins (recorded as the samples obtained by affinity purification) were collected.

The samples obtained by affinity purification were diluted with MMLV reverse transcriptase desalting diluent (50 mM Tris, 5% (V/V) glycerol, the balance was water, pH 7.5) at a ratio of 1:3.33 and then subjected to anion exchange chromatography. The specific steps were as follows.

According to the AKTA operating procedures, the working pump and system were rinsed with filtered and degassed MillQ water and connected with pre-packed HiTrap Q FF 5 mL at a flow rate of 0.5 mL/min. The column was rinsed with 5CV of $H_2O$ and then equilibrated with 5CV of MMLV reverse transcriptase A ionic solution (50 mM Tris, 150 mM NaCl, 5% (V/V) glycerol, the balance was water, pH 7.5). Then, the diluted samples were loaded onto the chromatographic column at 5 mL/min to collect the flow-through.

The flow-through obtained by the above anion exchange chromatography was diluted with MMLV reverse transcriptase desalting diluent at a ratio of 1:3 and then subjected to cation exchange chromatography. The specific steps were as follows.

According to the AKTA operating procedures, the working pump and system were washed with filtered and degassed MillQ water and connected with pre-packed column HiTrap SP FF 5 mL at a flow rate of 0.5 mL/min. The column was washed with 5CV of $H_2O$ and then equilibrated with 5CV of MMLV reverse transcriptase B ionic solution (50 mM Tris, 50 mM NaCl, 5% (V/V) glycerol, the balance was water, pH 7.5). Then, the diluted samples were loaded onto the chromatographic column at 5 mL/min. After loading, the column was rinsed with 5CV of MMLV reverse transcriptase B ionic solution, then eluted with a linear gradient of 0-100% (10CV) of MMLV reverse transcriptase C ionic solution (50 mM Tris, 1 M NaCl, 5% (V/V) glycerol, the balance was water, pH 7.5) and the target proteins, namely the purified target proteins, were collected.

Each purified target protein was dialyzed with MMLV reverse transcriptase stock solution (20 mM Tris, 100 mM NaCl, 1 mM DTT, 0.01% (V/V) NP-40, 0.1 mM EDTA, 50% Glycerol, the balance was water, pH 7.5), diluted to 1 mg/mL, and stored at -20°C to obtain fine-purified wild-type MMLV reverse transcriptase and various mutants, respectively.

**Example 3. Screen of target MMLV reverse transcriptase mutants by template switching performance assay**

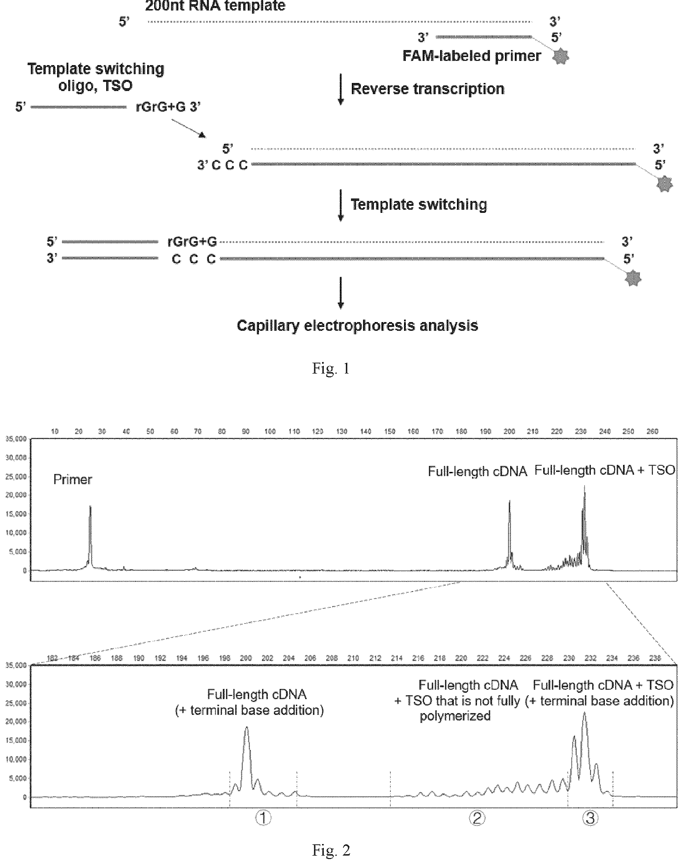

The method for screening the template switching performance of MMLV reverse transcriptase mutants is shown in Fig. 1. First, 200nt RNA synthesized in vitro was reverse transcribed using a primer with a FAM modification at the 5' end under the action of reverse transcriptase. CCC were added at the 3' end to generate a cDNA with a C tail. The CCC at the 3' end of the cDNA was complementary with the rGrG+G on template switching oligo (TSO). Under the template switching performance of the reverse transcriptase, the reaction was switched from the original use of RNA as a template to the use of TSO as a template for further extension to generate a template switching product of 230bp in length. Due to the FAM modification at the 5' end of the primer, the size and yield of the product can be determined by capillary electrophoresis, and the template switching efficiency of the wild-type MMLV reverse transcriptase and its mutants can be obtained by calculation.

Specifically, 25 ng/μL wild-type MMLV reverse transcriptase or each mutant shown in Table 1 were reacted at 42°C for 1 h in the following system, and incubated at 85°C for 15 min for inactivation.

System: 1x First Strand Buffer (BGI), 1 mM dNTPs, 5 mM DTT, 2 U/μL RNase Inhibitor (BGI), 10 μM TSO primer, 25 ng/μL 200nt Orf206b RNA transcribed in vitro, 0.5 μM FAM-Orf206b primer, 25 ng/μL wild-type MMLV reverse transcriptase or each mutant shown in Table 1, and the balance was water.

The information on the primers used was as follows:

TSO (SEQ ID NO. 3): 5' AAGCAGTGGTATCAACGCAGAGTACATrGrG+G 3', where rG represented riboguanosine; and +G represented LNA modified guanosine;
FAM-Orf206b (SEQ ID NO. 4): 5' GTGCTTACTTCTTCTTTTTGACCTACA 3'.

The sequence information of 200nt Orf206b RNA was as follows (SEQ ID NO. 5): GGGAUGUUGGAGUUU AGUGUUAUUGAAAGAGGCGGGUAUAUUCCUGCAGUAG AAAAAAAUAAGGCAUUCCUACGAGCAGAUGGUUG GAAUGACUAUUCCUUUGU UACAAUGUUUUAUCUUACUGUCUUUGAUGAGCAUGGUGAAAAAUGCGAUAUC GGAAAUGUUAAAAUUGGUUUUGUAGGUCAAAAGAAGAAGUAAGCAC.

Fig. 2 shows the capillary electrophoresis analysis of the reaction product, and the lower panel is an enlarged view of a part of the upper panel, where ① represents the reverse transcribed full-length cDNA; ② represents the product of the reverse transcribed full-length cDNA that undergoes template switching but is not fully polymerized; ③ represents the product of the reverse transcribed full-length cDNA that undergoes template switching and is fully polymerized.

The template switching efficiency of the MMLV reverse transcriptase and its mutants was calculated according to the following formula, so as to further obtain the strength of the template switching performance of the MMLV reverse transcriptase mutants compared with the wild-type reverse transcriptase. The results are shown in Table 2.

$$\text{Template switching efficiency} = (②+③) / (①+②+③).$$

The higher the template switching efficiency, the higher the template switching activity (performance) of reverse transcriptase.

In Table 2 below, relative activity = template switching efficiency of mutant / template switching efficiency of wild-type MMLV reverse transcriptase.

Table 2. Template switching activity of MMLV reverse transcriptase mutants compared with wild-type reverse transcriptase

| Mutant name | Template switching efficiency | Relative activity | Mutant name | Template switching efficiency | Relative activity |
|---|---|---|---|---|---|
| Clone 1 | 3.68% | 2.51 | Clone 46 | 4.64% | 3.16 |
| Clone 2 | 1.11% | 0.75 | Clone 47 | 5.66% | 3.85 |
| Clone 3 | 2.28% | 1.55 | Clone 48 | 5.66% | 3.85 |
| Clone 4 | 0.99% | 0.68 | Clone 49 | 7.17% | 4.88 |
| Clone 5 | 1.48% | 1.01 | Clone 50 | 2.55% | 1.73 |
| Clone 6 | 9.70% | 6.60 | Clone 51 | 14.47% | 9.84 |
| Clone 7 | 0.65% | 0.44 | Clone 52 | 7.44% | 5.06 |

(continued)

| Mutant name | Template switching efficiency | Relative activity | Mutant name | Template switching efficiency | Relative activity |
|---|---|---|---|---|---|
| Clone 8 | 4.00% | 2.72 | Clone 53 | 2.99% | 2.03 |
| Clone 9 | 2.14% | 1.46 | Clone 54 | 6.78% | 4.61 |
| Clone 10 | 1.08% | 0.74 | Clone 55 | 5.42% | 3.69 |
| Clone 11 | 1.93% | 1.31 | Clone 56 | 6.67% | 4.54 |
| Clone 12 | 0.38% | 0.26 | Clone 57 | 45.57% | 31.00 |
| Clone 13 | 8.29% | 5.64 | Clone 58 | 15.55% | 10.58 |
| Clone 14 | 3.24% | 2.20 | Clone 59 | 47.94% | 32.61 |
| Clone 15 | 0.93% | 0.63 | Clone 60 | 24.13% | 16.41 |
| Clone 16 | 3.00% | 2.04 | Clone 61 | 25.25% | 17.18 |
| Clone 17 | 1.88% | 1.28 | Clone 62 | 34.15% | 23.23 |
| Clone 18 | 6.46% | 4.39 | Clone 63 | 11.91% | 8.10 |
| Clone 19 | 0.45% | 0.31 | Clone 64 | 59.73% | 40.63 |
| Clone 20 | 0.00% | 0.00 | Clone 65 | 40.59% | 27.61 |
| Clone 21 | 11.84% | 8.05 | Clone 66 | 48.68% | 33.12 |
| Clone 22 | 2.21% | 1.50 | Clone 67 | 49.29% | 33.53 |
| Clone 23 | 2.37% | 1.61 | Clone 68 | 59.96% | 40.79 |
| Clone 24 | 5.87% | 3.99 | Clone 69 | 61.12% | 41.58 |
| Clone 25 | 0.53% | 0.36 | Clone 70 | 30.18% | 20.53 |
| Clone 26 | 2.27% | 1.54 | Clone 71 | 47.77% | 32.50 |
| Clone 27 | 1.06% | 0.72 | Clone 72 | 48.63% | 33.08 |
| Clone 28 | 1.45% | 0.98 | Clone 73 | 59.16% | 40.24 |
| Clone 29 | 6.55% | 4.46 | Clone 74 | 54.60% | 37.14 |
| Clone 30 | 4.30% | 2.93 | Clone 75 | 62.38% | 42.44 |
| Clone 31 | 5.79% | 3.94 | Clone 76 | 30.20% | 20.54 |
| Clone 32 | 3.43% | 2.33 | Clone 77 | 52.44% | 35.67 |
| Clone 33 | 1.60% | 1.09 | Clone 78 | 55.63% | 37.84 |
| Clone 34 | 1.35% | 0.92 | Clone 79 | 50.44% | 34.31 |
| Clone 35 | 2.90% | 1.98 | Clone 80 | 18.42% | 12.53 |
| Clone 36 | 5.44% | 3.70 | Clone 81 | 7.22% | 4.91 |
| Clone 37 | 8.19% | 5.57 | Clone 82 | 14.44% | 9.82 |
| Clone 38 | 3.63% | 2.47 | Clone 83 | 6.67% | 4.54 |
| Clone 39 | 1.20% | 0.81 | Clone 84 | 17.39% | 11.83 |
| Clone 40 | 2.37% | 1.61 | Clone 85 | 48.01% | 32.66 |
| Clone 41 | 4.33% | 2.95 | Clone 86 | 32.61% | 22.18 |
| Clone 42 | 3.69% | 2.51 | Clone 87 | 35.0% | 23.8 |
| Clone 43 | 4.98% | 3.39 | **WT** | **1.47%** | **1.00** |
| Clone 44 | 10.98% | 7.47 | SSII | 4.65% | 3.16 |
| Clone 45 | 1.92% | 1.31 | | | |

From Table 2, MMLV reverse transcriptase mutants with template switching performance increased to 1 to 42 times that of the wild-type MMLV reverse transcriptase were screened as target MMLV reverse transcriptase mutants.

**Example 4. Detection of the non-templated base addition performance of MMLV reverse transcriptase and its mutants**

The non-templated base addition performance of wild-type MMLV reverse transcriptase and each mutant shown in Table 1 was detected by using an RNA/DNA hybrid strand as a substrate, in which the 5' end of DNA in the hybrid strand was modified with FAM, and non-templated bases could be added at the 3' end of the DNA strand under the action of reverse transcriptase. Due to the FAM modification at the 5' end of the primer, the size and yield of the product can be determined by capillary electrophoresis, and the non-templated base addition efficiency of the MMLV reverse transcriptase and its mutants can be obtained by calculation.

Specifically, 25 ng/μL wild-type MMLV reverse transcriptase or its mutants shown in Table 1 were reacted at 42°C for 1 h in the following system and then treated at 85°C for 15 min for inactivation.

System: 1x First Strand Buffer, 10 mM DTT, 1 mM dNTPs, 5 ng/μL RNA70F, 0.5 μM FAM-DNA70R, 2 U/μL RNase Inhibitor, 25 ng/μL wild-type MMLV reverse transcriptase or its mutants shown in Table 1, the balance was water.

The information on the primers used was as follows:

RNA70F (SEQ ID NO. 6): 5'

AAUGAUACGGCGACCACCGAGAUCUACACUAGAUCGCUCGUCGGCAGCGUCAG

AUGUGUAUAAG AGACAG 3'

FAM-DNA70R (SEQ ID NO. 7): 5' FAM-

TGTCTCTTATACACATCTGACGCTGCCGACGAGCGATCTAGTGTAGATCTCGGTGGT

CGCCGTATCATT 3'

The products after the reaction were analyzed by capillary electrophoresis. The non-templated base addition efficiency of the wild-type MMLV reverse transcriptase and its mutants was calculated based on the "ratio of the sum of peak heights (or peak areas) greater than 70bp to the sum of peak areas of all bands" (reflecting the non-templated base addition activity, the higher the ratio, the higher the activity), so as to obtain the strength of the non-templated base addition performance of the MMLV reverse transcriptase mutants compared with the wild-type reverse transcriptase. The results are shown in Table 3.

In Table 3 below, relative activity = non-templated base addition efficiency of mutant / non-templated base addition efficiency of wild-type MMLV reverse transcriptase.

Table 3. Non-templated base addition activity of MMLV reverse transcriptase mutants compared with wild-type reverse transcriptase

| Mutant name | Non-templated addition efficiency | Relative activity | Mutant name | Non-templated addition efficiency | Relative activity |
|---|---|---|---|---|---|
| Clone 1 | 50.31% | 1.40 | Clone 46 | 49.49% | 1.38 |
| Clone 2 | 40.21% | 1.12 | Clone 47 | 52.15% | 1.45 |
| Clone 3 | 35.56% | 0.99 | Clone 48 | 56.92% | 1.58 |
| Clone 4 | 33.73% | 0.94 | Clone 49 | 48.91% | 1.36 |
| Clone 5 | 31.68% | 0.88 | Clone 50 | 49.07% | 1.36 |
| Clone 6 | 36.92% | 1.03 | Clone 51 | 41.22% | 1.15 |
| Clone 7 | 35.34% | 0.98 | Clone 52 | 48.74% | 1.36 |
| Clone 8 | 37.82% | 1.05 | Clone 53 | 34.64% | 0.96 |

(continued)

| Mutant name | Non-templated addition efficiency | Relative activity | Mutant name | Non-templated addition efficiency | Relative activity |
|---|---|---|---|---|---|
| Clone 9 | 37.04% | 1.03 | Clone 54 | 56.80% | 1.58 |
| Clone 10 | 32.17% | 0.89 | Clone 55 | 49.85% | 1.39 |
| Clone 11 | 36.87% | 1.03 | Clone 56 | 35.04% | 0.97 |
| Clone 12 | 24.30% | 0.68 | Clone 57 | 62.58% | 1.74 |
| Clone 13 | 35.21% | 0.98 | Clone 58 | 50.67% | 1.41 |
| Clone 14 | 39.76% | 1.11 | Clone 59 | 57.09% | 1.59 |
| Clone 15 | 30.52% | 0.85 | Clone 60 | 50.76% | 1.41 |
| Clone 16 | 28.52% | 0.79 | Clone 61 | 66.39% | 1.85 |
| Clone 17 | 52.57% | 1.46 | Clone 62 | 46.37% | 1.29 |
| Clone 18 | 41.62% | 1.16 | Clone 63 | 34.17% | 0.95 |
| Clone 19 | 39.86% | 1.11 | Clone 64 | 61.86% | 1.72 |
| Clone 20 | 24.41% | 0.68 | Clone 65 | 51.62% | 1.44 |
| Clone 21 | 54.04% | 1.50 | Clone 66 | 60.21% | 1.67 |
| Clone 22 | 36.86% | 1.03 | Clone 67 | 54.93% | 1.53 |
| Clone 23 | 34.54% | 0.96 | Clone 68 | 57.54% | 1.60 |
| Clone 24 | 36.70% | 1.02 | Clone 69 | 54.74% | 1.52 |
| Clone 25 | 25.90% | 0.72 | Clone 70 | 52.16% | 1.45 |
| Clone 26 | 28.05% | 0.78 | Clone 71 | 55.95% | 1.56 |
| Clone 27 | 29.00% | 0.81 | Clone 72 | 55.97% | 1.56 |
| Clone 28 | 32.26% | 0.90 | Clone 73 | 58.58% | 1.63 |
| Clone 29 | 53.31% | 1.48 | Clone 74 | 49.80% | 1.39 |
| Clone 30 | 33.26% | 0.93 | Clone 75 | 55.21% | 1.54 |
| Clone 31 | 45.34% | 1.26 | Clone 76 | 54.23% | 1.51 |
| Clone 32 | 40.45% | 1.13 | Clone 77 | 57.83% | 1.61 |
| Clone 33 | 26.53% | 0.74 | Clone 78 | 58.80% | 1.64 |
| Clone 34 | 34.00% | 0.95 | Clone 79 | 45.30% | 1.26 |
| Clone 35 | 44.31% | 1.23 | Clone 80 | 31.33% | 0.87 |
| Clone 36 | 45.09% | 1.25 | Clone 81 | 30.13% | 0.84 |
| Clone 37 | 53.28% | 1.48 | Clone 82 | 31.47% | 0.88 |
| Clone 38 | 35.31% | 0.98 | Clone 83 | 34.01% | 0.95 |
| Clone 39 | 28.22% | 0.78 | Clone 84 | 38.37% | 1.07 |
| Clone 40 | 27.72% | 0.77 | Clone 85 | 32.05% | 0.89 |
| Clone 41 | 51.11% | 1.42 | Clone 86 | 36.01% | 1.00 |
| Clone 42 | 49.78% | 1.38 | Clone 87 | / | / |
| Clone 43 | 35.70% | 0.99 | WT | 35.95% | 1.00 |
| Clone 44 | 33.55% | 0.93 | | | |
| Clone 45 | 49.94% | 1.39 | | | |

These results showed that the non-templated base addition activities of the screened target MMLV reverse transcriptase mutants were higher or lower compared with the non-templated base addition activity of the wild-type MMLV reverse transcriptase. Moreover, the non-templated base addition activities of some of the target MMLV reverse transcriptase mutants were increased to 1 to 1.85 times that of the wild-type reverse transcriptase.

**Example 5. Detection of the polymerization activity of MMLV reverse transcriptase and its mutants**

The activity of wild-type MMLV reverse transcriptase and its mutants was detected by using Oligo $(dT)_{16}$ as a primer and Poly $(rA)_{50}$ as a template, with strand extension occurring in the presence of reverse transcriptase to obtain an extended hybrid strand of RNA and cDNA. The fluorescent molecules were incorporated into the reaction product, and the activity of the reverse transcriptase was calculated by detecting the amount of RNA/cDNA product.

The specific reaction system was: 1x First Strand Buffer, 10 mM DTT, 0.5 mM dTTP, 1.5 mM Oligo $(dT)_{16}$, 25 ng/$\mu$L Poly $(rA)_{50}$, 2 U/$\mu$L RNase Inhibitor, 0.1 ng/$\mu$L wild-type MMLV reverse transcriptase or its mutants, the balance was water.

The above reaction system was reacted at 37°C for 10 min. After the reaction was terminated by adding 25 mM EDTA, 2 $\mu$L of the reaction product was taken for quantified using the Qubit dsDNA HS Assay Kit. The polymerization activity of the MMLV reverse transcriptase mutants compared with the wild-type MMLV reverse transcriptase was calculated. The results are shown in Table 4.

In Table 4, relative activity = dsDNA concentration of mutant / dsDNA concentration of wild-type MMLV reverse transcriptase, and the dsDNA concentrations shown in the table have been background-subtracted (dsDNA concentration of an enzyme-free control group).

Table 4. Polymerization activity of MMLV reverse transcriptase mutants compared with wild-type reverse transcriptase

| Mutant name | dsDNA concentration (ng/uL) | Relative activity | Mutant name | dsDNA concentration (ng/uL) | Relative activity |
|---|---|---|---|---|---|
| Clone 1 | 24.19 | 2.26 | Clone 46 | 9.19 | 0.86 |
| Clone 2 | 15.59 | 1.46 | Clone 47 | 10.39 | 0.97 |
| Clone 3 | 15.29 | 1.43 | Clone 48 | 11.19 | 1.05 |
| Clone 4 | 15.19 | 1.42 | Clone 49 | 17.99 | 1.68 |
| Clone 5 | 17.19 | 1.61 | Clone 50 | 11.99 | 1.12 |
| Clone 6 | 12.49 | 1.17 | Clone 51 | 11.19 | 1.05 |
| Clone 7 | 13.39 | 1.25 | Clone 52 | 12.99 | 1.22 |
| Clone 8 | 14.59 | 1.36 | Clone 53 | 14.29 | 1.34 |
| Clone 9 | 11.89 | 1.11 | Clone 54 | 8.99 | 0.84 |
| Clone 10 | 10.89 | 1.02 | Clone 55 | 14.39 | 1.35 |
| Clone 11 | 13.39 | 1.25 | Clone 56 | 11.59 | 1.08 |
| Clone 12 | 1.82 | 0.17 | Clone 57 | 8.59 | 0.80 |
| Clone 13 | 12.29 | 1.15 | Clone 58 | 4.19 | 0.39 |
| Clone 14 | 18.79 | 1.76 | Clone 59 | 7.19 | 0.67 |
| Clone 15 | 9.79 | 0.92 | Clone 60 | 6.89 | 0.64 |
| Clone 16 | 1.69 | 0.16 | Clone 61 | 10.59 | 0.99 |
| Clone 17 | 19.99 | 1.87 | Clone 62 | 5.59 | 0.52 |
| Clone 18 | 18.39 | 1.72 | Clone 63 | 3.99 | 0.37 |
| Clone 19 | 8.49 | 0.79 | Clone 64 | 3.89 | 0.36 |
| Clone 20 | 0.62 | 0.06 | Clone 65 | 6.09 | 0.57 |
| Clone 21 | 13.79 | 1.29 | Clone 66 | 9.79 | 0.92 |
| Clone 22 | 15.59 | 1.46 | Clone 67 | 7.29 | 0.68 |
| Clone 23 | 15.09 | 1.41 | Clone 68 | 10.39 | 0.97 |

(continued)

| Mutant name | dsDNA concentration (ng/uL) | Relative activity | Mutant name | dsDNA concentration (ng/uL) | Relative activity |
| --- | --- | --- | --- | --- | --- |
| Clone 24 | 17.59 | 1.65 | Clone 69 | 9.09 | 0.85 |
| Clone 25 | 11.29 | 1.06 | Clone 70 | 8.49 | 0.79 |
| Clone 26 | 0.42 | 0.04 | Clone 71 | 9.99 | 0.93 |
| Clone 27 | 8.49 | 0.79 | Clone 72 | 11.59 | 1.08 |
| Clone 28 | 8.79 | 0.82 | Clone 73 | 10.79 | 1.01 |
| Clone 29 | 14.99 | 1.40 | Clone 74 | 7.39 | 0.69 |
| Clone 30 | 10.79 | 1.01 | Clone 75 | 9.19 | 0.86 |
| Clone 31 | 22.29 | 2.09 | Clone 76 | 5.69 | 0.53 |
| Clone 32 | 13.59 | 1.27 | Clone 77 | 7.99 | 0.75 |
| Clone 33 | 4.59 | 0.43 | Clone 78 | 6.59 | 0.62 |
| Clone 34 | 7.89 | 0.74 | Clone 79 | 3.16 | 0.30 |
| Clone 35 | 16.19 | 1.51 | Clone 80 | 4.99 | 0.47 |
| Clone 36 | 12.99 | 1.22 | Clone 81 | 3.99 | 0.37 |
| Clone 37 | 18.99 | 1.78 | Clone 82 | 4.79 | 0.45 |
| Clone 38 | 16.69 | 1.56 | Clone 83 | 4.59 | 0.43 |
| Clone 39 | 17.79 | 1.66 | Clone 84 | 4.39 | 0.41 |
| Clone 40 | 13.99 | 1.31 | Clone 85 | 4.19 | 0.39 |
| Clone 41 | 14.29 | 1.34 | Clone 86 | 4.29 | 0.40 |
| Clone 42 | 10.59 | 0.99 | Clone 87 | 8.34 | 0.78 |
| Clone 43 | 10.59 | 0.99 | WT | 10.69 | 1.00 |
| Clone 44 | 9.79 | 0.92 | SSII | 12.40 | 1.16 |
| Clone 45 | 13.59 | 1.27 | | | |

As can be seen from Table 4, the polymerization activities of the screened target MMLV reverse transcriptase mutants were higher or lower compared with the wild-type MMLV reverse transcriptase.

**Example 6. Detection of the thermal stability of MMLV reverse transcriptase and its mutants**

The thermal stability of wild-type MMLV reverse transcriptase and various target mutants was detected by using the Protein Thermal Shift™ dye kit, which utilizes dyes bound to exposed hydrophobic residues to monitor the thermal stability of proteins by a real-time PCR instrument. The specific usage was based on the instructions of the Protein Thermal Shift™ dye kit.

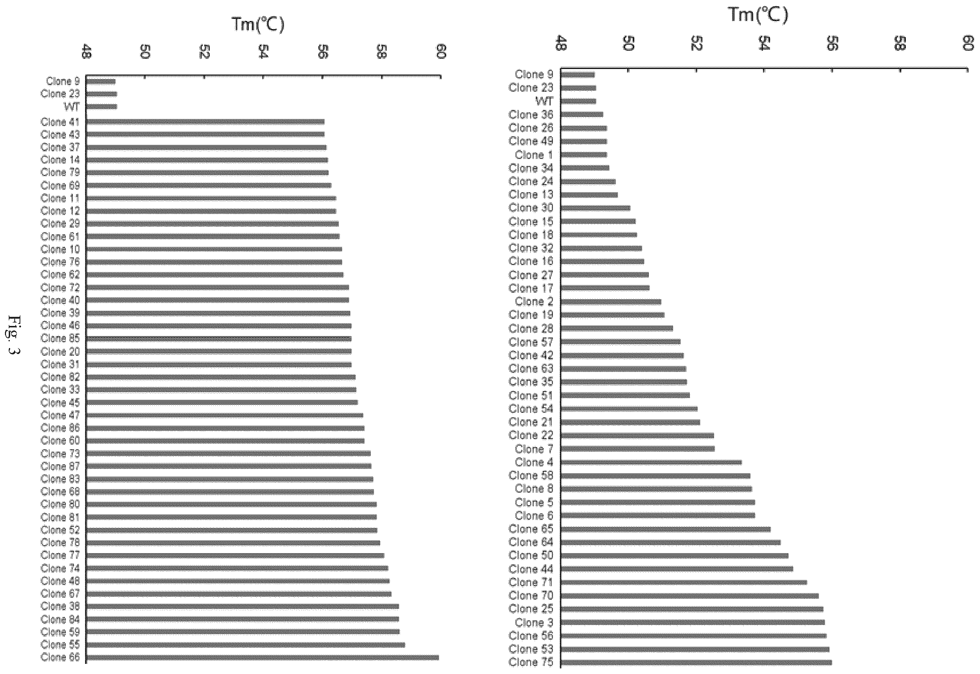

The detection results of the thermal stability of the MMLV reverse transcriptase and its mutants are shown in Fig. 3. As can be seen from Fig. 3, the thermal stability of most of the MMLV mutants was improved compared with the wild type.

**Example 7. Application of MMLV reverse transcriptase and its mutants in template-switching-based library construction**

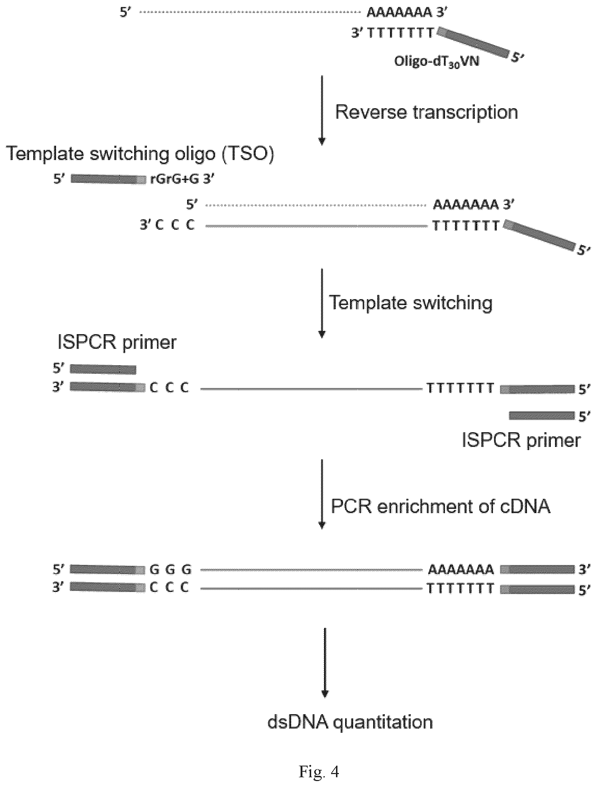

MMLV reverse transcriptase mutants were evaluated and screened using template-switching-based library construction methods such as Smart-Seq (Fig. 4). Specifically, mRNA with a poly A tail was reverse transcribed to synthesize a cDNA using an Oligo-dT$_{30}$VN primer. Due to the Cap structure at the 5' end of mRNA and the terminal transferase performance of the MMLV reverse transcriptase, non-template CCC bases were added to the 3' end the cDNA strand when it was extended to the end of mRNA. The CCC at the 3' end of the cDNA was complementary to the rGrG+G in template switching oligo (TSO). Under the template switching performance of the reverse transcriptase, the reaction was switched from the original use of mRNA as a template to the use of TSO as a template for extension to complete the first cDNA strand synthesis. Since Oligo-dT$_{30}$VN and TSO had partially identical sequences, cDNA can be enriched by PCR with ISPCR primers and amplified to ng level. The first cDNA strand synthesis reaction system was consisted of 1x First Strand Buffer, 1 mM dNTPs, 5 mM DTT, 1 M Betaine, 6 mM MgCl$_2$, 1 μM TSO primer, 1 μM Oligo-dT$_{30}$VN primer, 1 U/μL RNase Inhibitor, 1 ng/μL HEK293T RNA (obtained by extracting the RNA from HEK293T cells), and 10 ng/μL wild-type MMLV reverse transcriptase or mutants. The reaction was carried out at 42°C for 1 h to obtain a first cDNA strand synthesis product. Then, 5 μL of the first cDNA strand synthesis product, 0.25 μL of 10 μM ISPCR primer, and 12.5 μL of KAPA HiFi HotStart Ready Mix were added to 25 μL of the reaction system for PCR enrichment. The PCR program was as follows: pre-denaturation at 95°C for 5 min, 15 cycles of denaturation at 98°C for 20 s, annealing at 58°C for 20 s, extension at 72°C for 3 min, and final extension at 72°C for 5 min. The enriched cDNA was quantified using Qubit dsDNA HS Assay Kit, and the application effect of the reverse transcriptase mutants in the template-switching-based library construction methods was evaluated by the amount of dsDNA. The results are shown in Table 5.

In Table 5, relative activity = dsDNA concentration of each mutant / dsDNA concentration of wild-type MMLV reverse transcriptase. The dsDNA concentration shown in the table has had the background subtracted (dsDNA concentration of an enzyme-free control group).

The primer sequences used were as follows:

TSO primer (SEQ ID NO. 3): 5' AAGCAGTGGTATCAACGCAGAGTACATrGrG+G 3', where rG is ribonucleotide G, and +G is locked nucleotide G;

Oligo-dT$_{30}$VN primer (SEQ ID NO. 8): 5' AAGCAGTGGTATCAACGCAGAGTACTTTTTTTTTTTTTTTTTTTTTTTTTTTTTTVN 3', where V is a or g or c, and N is a or g or c or t;

ISPCR primer (SEQ ID NO. 9): 5' AAGCAGTGGTATCAACGCAGAGT 3'.

Table 5. dsDNA quantitative results for Smart-Seq reaction using MMLV reverse transcriptase mutants

| Mutant name | dsDNA concentration (ng/uL) | Relative activity | Mutant name | dsDNA concentration (ng/uL) | Relative activity |
|---|---|---|---|---|---|
| Clone 1 | 2.93 | 15.42 | Clone 46 | 1.16 | 6.11 |
| Clone 2 | 1.73 | 9.11 | Clone 47 | 2.24 | 11.79 |
| Clone 3 | 1.27 | 6.68 | Clone 48 | 4.97 | 26.16 |
| Clone 4 | 1.06 | 5.58 | Clone 49 | 2.36 | 12.42 |
| Clone 5 | 1.14 | 6.00 | Clone 50 | 1.68 | 8.84 |
| Clone 6 | 1.10 | 5.79 | Clone 51 | 7.81 | 41.11 |
| Clone 7 | 0.65 | 3.42 | Clone 52 | 4.65 | 24.47 |
| Clone 8 | 1.80 | 9.47 | Clone 53 | 0.92 | 4.84 |
| Clone 9 | 0.63 | 3.32 | Clone 54 | 2.70 | 14.21 |
| Clone 10 | 0.76 | 4.00 | Clone 55 | 3.14 | 16.53 |
| Clone 11 | 0.82 | 4.32 | Clone 56 | 1.36 | 7.16 |
| Clone 12 | 1.61 | 8.47 | Clone 57 | 23.07 | 121.39 |
| Clone 13 | 1.88 | 9.89 | Clone 58 | 14.08 | 74.12 |
| Clone 14 | 1.08 | 5.68 | Clone 59 | 28.13 | 148.03 |
| Clone 15 | 1.64 | 8.63 | Clone 60 | 13.94 | 73.37 |
| Clone 16 | 0.89 | 4.68 | Clone 61 | 13.87 | 72.99 |
| Clone 17 | 1.54 | 8.11 | Clone 62 | 13.80 | 72.62 |
| Clone 18 | 1.10 | 5.79 | Clone 63 | 8.17 | 42.98 |
| Clone 19 | 1.24 | 6.53 | Clone 64 | 18.79 | 98.88 |
| Clone 20 | 0.75 | 3.95 | Clone 65 | 13.30 | 69.99 |

(continued)

| Mutant name | dsDNA concentration (ng/uL) | Relative activity | Mutant name | dsDNA concentration (ng/uL) | Relative activity |
|---|---|---|---|---|---|
| Clone 21 | 2.61 | 13.74 | Clone 66 | 15.94 | 83.88 |
| Clone 22 | 1.65 | 8.68 | Clone 67 | 19.64 | 103.39 |
| Clone 23 | 1.02 | 5.37 | Clone 68 | 22.14 | 116.52 |
| Clone 24 | 2.73 | 14.37 | Clone 69 | 21.50 | 113.14 |
| Clone 25 | 0.84 | 4.42 | Clone 70 | 27.77 | 146.16 |
| Clone 26 | 0.94 | 4.95 | Clone 71 | 23.56 | 124.02 |
| Clone 27 | 0.75 | 3.95 | Clone 72 | 21.00 | 110.51 |
| Clone 28 | 0.87 | 4.58 | Clone 73 | 22.49 | 118.39 |
| Clone 29 | 5.83 | 30.68 | Clone 74 | 19.57 | 103.01 |
| Clone 30 | 2.65 | 13.95 | Clone 75 | 25.56 | 134.53 |
| Clone 31 | 1.49 | 7.84 | Clone 76 | 22.52 | 118.50 |
| Clone 32 | 1.13 | 5.95 | Clone 77 | 20.43 | 107.51 |
| Clone 33 | 0.78 | 4.11 | Clone 78 | 21.21 | 111.64 |
| Clone 34 | 0.84 | 4.42 | Clone 79 | 28.77 | 151.41 |
| Clone 35 | 1.75 | 9.21 | Clone 80 | 19.27 | 101.42 |
| Clone 36 | 1.85 | 9.74 | Clone 81 | 10.07 | 53.00 |
| Clone 37 | 4.70 | 24.74 | Clone 82 | 2.85 | 15.00 |
| Clone 38 | 1.16 | 6.11 | Clone 83 | 7.18 | 37.79 |
| Clone 39 | 1.12 | 5.89 | Clone 84 | 17.07 | 89.84 |
| Clone 40 | 1.17 | 6.16 | Clone 85 | 28.67 | 150.89 |
| Clone 41 | 1.35 | 7.11 | Clone 86 | 26.97 | 141.95 |
| Clone 42 | 0.88 | 4.63 | Clone 87 | 9.47 | 49.84 |
| Clone 43 | 1.18 | 6.21 | WT | 0.19 | 1.00 |
| Clone 44 | 3.11 | 16.37 | SSII | 4.08 | 21.47 |
| Clone 45 | 6.90 | 36.32 | | | |

Compared with the wild-type MMLV reverse transcriptase, the dsDNA yield of all mutants in the Smart-Seq library construction was increased to 1 to 150 times that of the wild type.

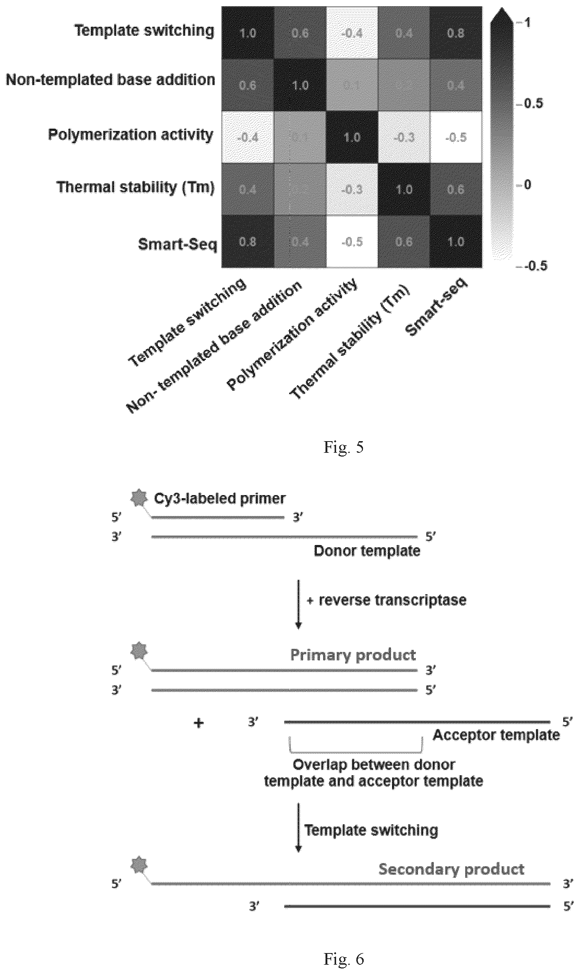

A correlation analysis was carried out between the dsDNA yield in the Smart-seq library construction and the template switching (TS), the non-templated base addition (NTA), the polymerization activity (Pol), and the thermal stability (Tm) of MMLV reverse transcriptase detected by the methods described in Examples 3 to 6 using data from 87 mutants including all mutants mentioned in the present disclosure and mutants not mentioned in the present disclosure with increased or unchanged Smart-Seq yield compared with the wild type. The analysis results are shown in Fig. 5. It can be seen that the polymerization activity was negatively correlated with the thermal stability, which was consistent with the common understanding that the improvement in thermal resistance is generally accompanied by the loss of activity in enzyme modification, indicating that the analysis results were relatively accurate and reliable.

Overall, the dsDNA yield of Smart-Seq was positively correlated with the template switching (TS), the non-templated addition (NTA), and the thermal stability (Tm), among which the template switching had the highest correlation with Smart-Seq. The polymerase activity of MMLV reverse transcriptase was essential for a reverse transcription process but was not critical in affecting the yield of the template-switching-based library preparation methods.

Therefore, template switching performance was selected as a criterion for screening target MMLV reverse transcriptase mutants, with auxiliary reference to non-templated base addition performance and thermal stability.

**Example 8. Verification of the template switching performance of fine-purified MMLV reverse transcriptase and its mutants**

The method for verifying the template switching performance of fine-purified MMLV reverse transcriptase and its mutants is shown in Fig. 6, that is, the polymerization reaction was carried out under the action of reverse transcriptase using a sequence with Cy3 at the 5' end as a primer and a donor template as a template to generate a primary product. The 5' sequence of the donor template overlapped with the 3' sequence of an acceptor template. Due to the template switching performance of the reverse transcriptase, it can be switched to use the acceptor template as a template to continue the polymerization reaction, generating a secondary product that is longer than the primary product. The amount of the secondary product can be used to determine the strength of the template switching performance of the reverse transcriptase. Specifically, the reaction was carried out in a reaction system, including 1x First Strand Buffer, 0.5 mM dNTPs, 0.5 $\mu$M Cy3-labeled primer, 0.5 $\mu$M donor template, 2.5 $\mu$M acceptor template, and 10 ng/$\mu$L MMLV reverse transcriptase or its mutants at 42°C for 1 h. The reaction was then terminated by adding 25 mM EDTA. 10 $\mu$L of the reaction product was taken and subjected to urea-polyacrylamide gel electrophoresis (Urea-PAGE) and photographed using a gel imaging device that can capture Cy3 fluorescence. The primer sequences used for detecting the template switching performance were as follows:

> Cy3-labeled primer: 5' Cy3-ATGATAAATGTCGTTAGTTTC 3' (SEQ ID NO. 10);
> Donor template: 5' CTGACGTCCTGCCACCGGAGAAACTAACGACATTTATCAT 3' (SEQ ID NO. 11);
> Acceptor template: 5' CATTAGCCAGAGCAAATATGCTGACGTCCTGCCACCGGA 3' (SEQ ID NO. 12).

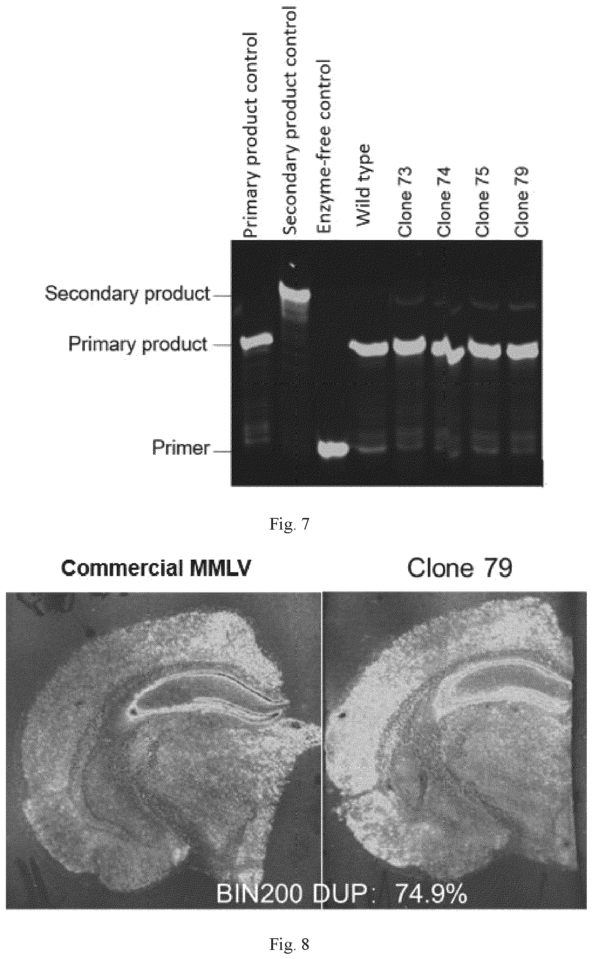

The detection results of the template switching of some fine-purified MMLV reverse transcriptase mutants are shown in Fig. 7, where WT is wild-type reverse transcriptase. As can be seen from Fig. 7, the band of secondary product (SP) could be detected for several MMLV reverse transcriptase mutants, while no band of secondary product (SP) was detected for the wild-type MMLV reverse transcriptase, indicating that the screened target MMLV reverse transcriptase mutants have improved template switching performance compared with the wild type.

**Example 9. Application test of fine-purified MMLV reverse transcriptase and its mutants in STOmics**

The Stomics test was performed using commercially available MMLV reverse transcriptase (Golden RT, purchased from BGI) and the modified and screened MMLV reverse transcriptase mutant Clone 79. The STOmics process included tissue patching, baking, formaldehyde fixation, ssDNA photography, tissue permeabilization, in situ reverse transcription, PCR amplification, library preparation, sequencing on the machine, and analysis. The sample used in this test was a mouse brain tissue section from Guangdong Medical Laboratory Animal Center. The sample was subject to tissue patching, baking, formaldehyde fixation, ssDNA photography, tissue permeabilization, in situ reverse transcription, and PCR amplification according to the method of Stero-seq transcriptome reagent kit (Cat. No. 101ST114, BGI). The PCR product was subsequently used to generate DNB (DNA nanoball) and finally sequenced using the DNBSEQ-Tx sequencer from MGI. The raw sequencing data was analyzed, and the visualization results were output.

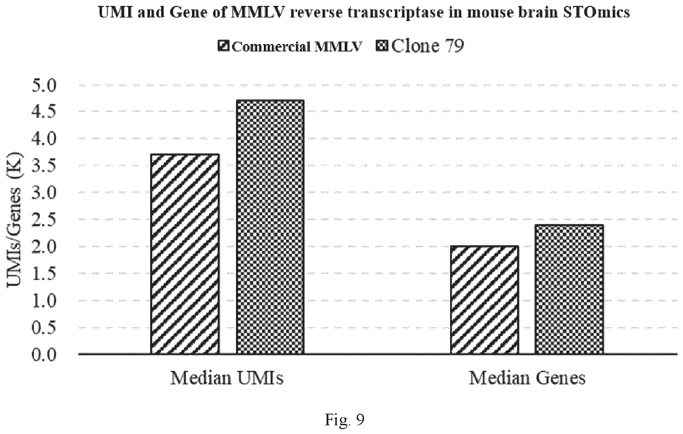

The visualization results of the commercially available MMLV reverse transcriptase and the modified and screened MMLV reverse transcriptase mutant Clone 79 are shown in Fig. 8. As can be seen from Fig. 8, the gene capture amount of the modified and screened MMLV reverse transcriptase mutant clone 79 was better than the commercially available MMLV reverse transcriptase. The comparative data of the probe-captured gene types (Median Gene Type) and the probe-captured UMI number (Median MID) for the commercially available MMLV reverse transcriptase and the modified and screened MMLV reverse transcriptase mutant Clone 79 are shown in Fig. 9. The Median Genes and Median UMIs of the modified and screened MMLV reverse transcriptase mutant Clone 79 were increased by 20% and 27%, respectively, compared with the commercially available MMLV reverse transcriptase.

**Industrial Applications**

The present disclosure screened out a variety of MMLV reverse transcriptase mutants with improved template switching performance by the template switching detection method (Fig. 1). In the template-switching-based library construction, the mutants with significantly higher product yields than wild-type MMLV reverse transcriptase were screened. In addition, the polymerization activity, the non-templated base addition, and the thermal stability of the screened MMLV reverse transcriptase mutants with improved template switching performance were further evaluated, some MMLV reverse transcriptase mutants also have improved thermal stability, non-templated base addition performance, or polymerization activity.

In summary, the present disclosure screened out the MMLV reverse transcriptase mutants with improved template switching performance compared with the wild type, which may have improved non-templated base addition performance, thermal stability, or polymerization activity. The screened mutants are suitable for RNA sequencing using template-switching-based library preparation methods, such as Smart-Seq, nanopore sequencing, and 5' RACE, and can also be applied to RT-qPCR and the like.

In summary, the present disclosure screened out reverse transcriptase mutants with improved template switching performance, which can be used for template-switching-based library preparation and RNA sequencing.

**Claims**

1. A Moloney murine leukemia virus, MMLV, reverse transcriptase mutant, having mutations at amino acid residue sites 66 and 68 compared with an amino acid sequence of a wild-type MMLV reverse transcriptase as set forth in SEQ ID NO. 2, the MMLV reverse transcriptase mutant retaining reverse transcriptase activity.

2. The mutant according to claim 1, wherein for the mutant:

   M at site 66 is mutated to V, K, Y, or L; and
   Q at site 68 is mutated to N or K.

3. The mutant according to claim 1 or 2, wherein one or more of the following amino acid residue sites of the mutant are mutated: 39, 62, 65, 67, 69, 70, 80, 81, 99, 105, 109, 116, 124, 152, 175, 176, 186, 200, 269, 284, 286, 289, 302, 306, 313, 333, 334, 425, 435, 450, 454, 524, 562, 583, and 653.

4. The mutant according to claim 3, wherein one or more mutated sites are respectively mutated in the way:

   M at site 39 is mutated to D or R;
   K at site 62 is mutated to R;
   P at site 65 is mutated to N, T, K, or L;
   S at site 67 is mutated to T;
   E at site 69 is mutated to K or R;
   A at site 70 is mutated to S;
   R at site 80 is mutated to T;
   L at site 81 is mutated to A;
   L at site 99 is mutated to A;
   G at site 105 is mutated to R;
   Y at site 109 is mutated to R;
   R at site 116 is mutated to D;
   D at site 124 is mutated to K;
   K at site 152 is mutated to R;
   P at site 175 is mutated to S;
   E at site 176 is mutated to R;
   T at site 186 is mutated to A;
   D at site 200 is mutated to N;
   L at site 269 is mutated to R;
   R at site 284 is mutated to T;
   E at site 286 is mutated to R;
   M at site 289 is mutated to L;
   E at site 302 is mutated to K;
   T at site 306 is mutated to R;
   W at site 313 is mutated to F;
   L at site 333 is mutated to A or K;
   F at site 334 is mutated to N;
   K at site 425 is mutated to R;
   L at site 435 is mutated to G;
   R at site 450 is mutated to H;
   N at site 454 is mutated to K;
   D at site 524 is mutated to G or A;
   E at site 562 is mutated to Q;
   D at site 583 is mutated to N;

D at site 653 is mutated to N.

5. The mutant according to any one of claims 1 to 4, wherein compared with the wild-type MMLV reverse transcriptase, the MMLV reverse transcriptase mutant has improved template switching activity, improved non-templated base addition performance, and/or improved thermal stability.

6. The mutant according to claim 5, wherein compared with the wild-type MMLV reverse transcriptase, the template switching activity of the MMLV reverse transcriptase mutant is increased to 1 to 42 times that of the wild-type MMLV reverse transcriptase.

7. The mutant according to claim 5, wherein compared with the wild-type MMLV reverse transcriptase, the non-templated base addition performance of the MMLV reverse transcriptase mutant is increased to 1 to 1.85 times that of the wild-type MMLV reverse transcriptase.

8. The mutant according to any one of claims 1 to 7, wherein the MMLV reverse transcriptase mutant has any one of the following mutations:

  M66L and Q68K; or
  M66V and Q68K; or
  M66V, S67T, and Q68K; or
  M66V, Q68K, and R80T; or
  M66V, Q68K, and D124K; or
  M66V, Q68K, and D200N; or
  M66V, Q68K, and T306K; or
  M66V, Q68K, and T306R; or
  M66V, Q68K, E176R, and L333A; or
  M66L, Q68K, E69K, E302K, W313F, and N454K; or
  M66V, Q68K, E69K, E302K, W313F, and N454K; or
  M66V, Q68K, E69K, E302K, T306R, W313F, and N454K; or
  M66L, Q68K, E69K, E302K, W313F, N454K, and D524G; or
  M66V, Q68K, E69K, E302K, W313F, N454K, and D524G; or
  M66L, Q68K, E69K, E302K, W313F, N454K, D524G, and D653N; or
  M66V, Q68K, E69K, E302K, W313F, N454K, D524G, and D653N; or
  M66V, Q68K, E69K, E302K, T306R, W313F, N454K, D524G, and D653N.

9. The mutant according to claim 6, wherein the MMLV reverse transcriptase mutant has any one of the following mutations:

  M66L and Q68K; or
  M66V and Q68K; or
  M66V, S67T, and Q68K; or
  M66V, Q68K, and R80T; or
  M66V, Q68K, and D124K; or
  M66V, Q68K, and D200N; or
  M66V, Q68K, and T306K; or
  M66V, Q68K, and T306R; or
  M66V, Q68K, E176R, and L333A; or
  M66L, Q68K, E69K, E302K, W313F, N454K, D524G, and D653N; or
  M66V, Q68K, E69K, E302K, W313F, N454K, D524G, and D653N; or
  M66V, Q68K, E69K, E302K, T306R, W313F, N454K, D524G, and D653N.

10. The mutant according to claim 6, wherein the MMLV reverse transcriptase mutant has any one of the following mutations:

  M66L and Q68K; or
  M66V and Q68K; or
  M66V, S67T, and Q68K; or
  M66V, Q68K, and D124K; or

M66V, Q68K, and D200N; or
M66V, Q68K, and T306R; or
M66V, Q68K, E176R, and L333A; or
M66L, Q68K, E69K, E302K, W313F, N454K, D524G, and D653N; or
M66V, Q68K, E69K, E302K, W313F, N454K, D524G, and D653N; or
M66V, Q68K, E69K, E302K, T306R, W313F, N454K, D524G, and D653N.

11. A nucleic acid molecule, encoding the mutant according to any one of claims 1 to 10.

12. An expression cassette, recombinant vector, or transgenic cell line comprising the nucleic acid molecule according to claim 11.

13. A recombinant bacterium, comprising the nucleic acid molecule according to claim 11.

14. Use of the nucleic acid molecule according to claim 11, or the expression cassette, recombinant vector, or transgenic cell line according to claim 12, or the recombinant bacterium according to claim 13 in the preparation of the MMLV reverse transcriptase.

15. A method for preparing the mutant according to any one of claims 1 to 10, the method comprising:

culturing the recombinant bacterium according to claim 13, and
performing an induction treatment to obtain the mutant according to any one of claims 1 to 10.

16. A kit, comprising the mutant according to any one of claims 1 to 10.

17. The kit according to claim 16, further comprising at least one of:

one or more nucleotides;
one or more DNA polymerases;
one or more primers, one or more primers; and
one or more terminators.

18. A method for reverse transcription of a nucleic acid molecule, the method comprising:

mixing at least one nucleic acid template with at least one reverse transcriptase to obtain a mixture, wherein the reverse transcriptase is the mutant according to any one of claims 1 to 10; and
performing a reverse transcription reaction of the mixture to obtain a reverse transcription nucleic acid molecule that is entirely or partially complementary to the at least one template.

19. A method for amplifying a nucleic acid, the method comprising:

performing a first mixing reaction by mixing at least one nucleic acid template with at least one reverse transcriptase, to obtain a reaction product, wherein the reverse transcriptase is the mutant according to any one of claims 1 to 10; and
performing a second mixing reaction by mixing the reaction product with at least one DNA polymerase, to obtain an amplified nucleic acid molecule.

20. A method for constructing a template-switching-based sequencing library, the method comprising:

1) extracting RNA from a biological sample to be tested and performing a reverse transcription by using the method according to claim 18, to obtain a cDNA; and
2) constructing a sequencing library based on the cDNA.

21. The method according to claim 20, wherein the biological sample to be tested is animal tissue, plant tissue, bacteria, or cells.

22. The method according to claim 20, wherein the biological sample to be tested is selected from at least one of soil, feces, blood, and serum.

**23.** The method according to claim 20, wherein the sequencing library is a high-throughput sequencing library.

Fig. 1

Fig. 2

Fig. 3

5′ ┄┄┄┄┄┄┄┄┄┄┄┄┄┄┄┄┄┄┄ AAAAAAA 3′
3′ TTTTTTT◤
Oligo-dT₃₀VN    *5′*

↓ Reverse transcription

Template switching oligo (TSO)
5′ ▬▬▬▬ rGrG+G 3′
5′ ┄┄┄┄┄┄┄┄┄┄┄┄┄┄┄┄┄┄┄┄┄┄┄ AAAAAAA 3′
3′ C C C ────────────────────── TTTTTTT◤
*5′*

↓ Template switching

ISPCR primer
5′ ▬▬▬▬
3′ ▬▬▬ C C C ─────────────────── TTTTTTT ▬▬▬ 5′
▬▬▬ 5′
ISPCR primer

↓ PCR enrichment of cDNA

5′ ▬▬▬ G G G ─────────────────── AAAAAAA ▬▬▬ 3′
3′ ▬▬▬ C C C ─────────────────── TTTTTTT ▬▬▬ 5′

↓

dsDNA quantitation

Fig. 4

Fig. 5

Fig. 6

Fig. 7

Fig. 8

UMI and Gene of MMLV reverse transcriptase in mouse brain STOmics

☑ Commercial MMLV  ☒ Clone 79

Fig. 9

## INTERNATIONAL SEARCH REPORT

| International application No. |
| --- |
| **PCT/CN2022/129839** |

| A. | CLASSIFICATION OF SUBJECT MATTER |
| --- | --- |

C12N9/12(2006.01)i; C12N15/63(2006.01)i; C12Q1/686(2018.01)i

According to International Patent Classification (IPC) or to both national classification and IPC

| B. | FIELDS SEARCHED |
| --- | --- |

Minimum documentation searched (classification system followed by classification symbols)

IPC: C12N, C12Q

Documentation searched other than minimum documentation to the extent that such documents are included in the fields searched

Electronic data base consulted during the international search (name of data base and, where practicable, search terms used)

CNABS, DWPI, WPABS, CNTXT, TWTXT, WOTXT, USTXT, EPTXT, JPTXT, ENTXT, CNKI, 万方数据库, WANFANG DATABASE, WEB OF SCIENCE, PUBMED: 逆转录酶, 突变体, reverse transcriptase, MMLV, mutant, M66L, M66V, Q68K; GENBANK+EMBL+; 中国专利生物序列检索系统, China Patents Biological Sequence Search System: 对 SEQ ID No: 2的检索: search conducted on SEQ ID NO: 2.

| C. | DOCUMENTS CONSIDERED TO BE RELEVANT |
| --- | --- |

| Category* | Citation of document, with indication, where appropriate, of the relevant passages | Relevant to claim No. |
| --- | --- | --- |
| A | CN 103348004 A (BIONEER CORP.) 09 October 2013 (2013-10-09) see claims 1-11 | 1-23 |
| A | CN 113174381 A (YEASEN BIOTECHNOLOGY (SHANGHAI) CO., LTD.) 27 July 2021 (2021-07-27) see claims 1-2 | 1-23 |
| A | CN 110291196 A (TAKARA BIO, INC.) 27 September 2019 (2019-09-27) see claims 1-17 | 1-23 |
| A | CN 112695019 A (YEASEN BIOTECHNOLOGY (SHANGHAI) CO., LTD.) 23 April 2021 (2021-04-23) see claims 1-7 | 1-23 |
| A | Shinnick, T. M. et al. "accession no. NP_955591: p80 RT [Moloney murine leukemia virus]" *GenBank Database*, 13 August 2018 (2018-08-13), see sequence and information | 1-23 |

☐ Further documents are listed in the continuation of Box C. ☑ See patent family annex.

| * | Special categories of cited documents: |
| --- | --- |
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
| --- | --- |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
| --- | --- |
| **04 July 2023** | **19 July 2023** |

| Name and mailing address of the ISA/CN | Authorized officer |
| --- | --- |
| **China National Intellectual Property Administration (ISA/ CN)** **China No. 6, Xitucheng Road, Jimenqiao, Haidian District, Beijing 100088** | |
| | Telephone No. |

Form PCT/ISA/210 (second sheet) (July 2022)

**INTERNATIONAL SEARCH REPORT**

International application No.

**PCT/CN2022/129839**

| Box No. I | Nucleotide and/or amino acid sequence(s) (Continuation of item 1.c of the first sheet) |

1. With regard to any nucleotide and/or amino acid sequence disclosed in the international application, the international search was carried out on the basis of a sequence listing:

    a. ☑ forming part of the international application as filed.

    b. ☐ furnished subsequent to the international filing date for the purposes of international search (Rule 13*ter*.1(a)),

        ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

**INTERNATIONAL SEARCH REPORT**
Information on patent family members

International application No.

**PCT/CN2022/129839**

| Patent document cited in search report | | | Publication date (day/month/year) | Patent family member(s) | | | Publication date (day/month/year) |
|---|---|---|---|---|---|---|---|
| CN | 103348004 | A | 09 October 2013 | EP | 2902486 | A1 | 05 August 2015 |
| | | | | EP | 2902486 | B1 | 27 September 2017 |
| | | | | WO | 2012108672 | A2 | 16 August 2012 |
| | | | | WO | 2012108672 | A9 | 01 November 2012 |
| | | | | WO | 2012108672 | A3 | 20 December 2012 |
| | | | | US | 2014045244 | A1 | 13 February 2014 |
| | | | | US | 9534210 | B2 | 03 January 2017 |
| | | | | KR | 20120091712 | A | 20 August 2012 |
| | | | | KR | 101818126 | B1 | 15 January 2018 |
| | | | | EP | 2673359 | A2 | 18 December 2013 |
| | | | | EP | 2673359 | A4 | 03 September 2014 |
| | | | | JP | 2014506462 | A | 17 March 2014 |
| | | | | JP | 5883036 | B2 | 09 March 2016 |
| CN | 113174381 | A | 27 July 2021 | None | | | |
| CN | 110291196 | A | 27 September 2019 | WO | 2018110595 | A1 | 21 June 2018 |
| | | | | KR | 20190091499 | A | 06 August 2019 |
| | | | | KR | 102481388 | B1 | 27 December 2022 |
| | | | | EP | 3556854 | A1 | 23 October 2019 |
| | | | | EP | 3556854 | A4 | 19 August 2020 |
| | | | | US | 2021277367 | A1 | 09 September 2021 |
| | | | | US | 11220677 | B2 | 11 January 2022 |
| | | | | JPWO | 2018110595 | A1 | 24 October 2019 |
| | | | | JP | 7061078 | B2 | 27 April 2022 |
| CN | 112695019 | A | 23 April 2021 | None | | | |

Form PCT/ISA/210 (patent family annex) (July 2022)